# EUROPEAN PATENT APPLICATION

(11) **EP 4 775 150 A1**
(43) Date of publication of application: **15.07.2026**
(21) Application number: 26150791.7
(22) Date of filing: 08.01.2026
(51) Int. Cl.: A61B 8/00

(54) **CHARACTERIZATION OF CONTRAST ENHANCED ULTRASOUND IMAGES**

(30) Priority: 13.01.2025 US 202519018503
(71) Applicant: Siemens Medical Solutions USA, Inc., Malvern, PA 19355 (US)
(72) Inventor: GOL GUNGOR, Derya, Palo Alto, CA, 94304-1038 (US)
(74) Representative: HKW Intellectual Property PartG mbB

(57) **Abstract**

A framework for characterizing contrast enhanced ultrasound (CEUS) images. The framework derives (204) a brightness mode (B-mode) and contrast image sequence from the sequence of contrast enhanced ultrasound (CEUS) images. B-mode images and contrast images in the B-mode and contrast image sequence are spatially co-registered and temporally sub-sampled. A region of interest may be selected (206) in a frame of the B-mode and contrast image sequence. The region of interest is then characterized (208) based on the B-mode and contrast image sequence.

## Description

### TECHNICAL FIELD

The present disclosure generally relates to medical imaging, and more particularly to a framework for characterizing contrast enhanced ultrasound images.

### BACKGROUND

Ultrasound imaging is a noninvasive medical test that helps physicians diagnose and treat medical conditions. Ultrasound imaging involves using a small probe called a transducer placed directly on the skin to transmit and receive highfrequency sound waves that travel into the body. The sound waves are used to create a medical diagnostic image of the internal structures of the body.

Abdominal contrast-enhanced ultrasound (CEUS) combines ultrasound of the abdomen with a special type of intravenous contrast agent to improve the visualization of blood vessels and organs. The contrast agent may include gas-filled microbubbles to better visualize organs and blood vessels within the abdomen and pelvis. Abdominal CEUS examination may evaluate the liver, spleen, kidneys, pancreas, bowel, and/or bladder for a variety of diseases and conditions.

CEUS may greatly improve the diagnostic accuracy of ultrasound in the detection and characterization of focal liver lesions. In Liver-CEUS scans, the aim is to determine the type of the focal liver lesions by injecting a contrast agent to the patient and observing wash-in and wash-out properties. Wash-in generally refers to the enhancement of a tissue or mass after contrast injection, and is seen in benign or non-cancerous tissues. Wash-out generally indicates that a tissue or mass loses contrast faster than normal liver tissue, and is seen in cancerous or malignant tissues. Since wash-in happens quite fast (e.g., within 20-30 seconds), a video may be saved to observe the wash-in process. Wash-out images may be acquired discretely, such as 1 minute apart from each other until around 5 mins. However, it may be difficult for the physician to track any lesions in the video and/or separate images.

In some applications, frames of data acquired over time are integrated. The resulting image may provide useful information for diagnosis, such as showing smaller vessels or perfusion channels. However, due to operator motion (e.g., sweeping motion) or internal motion (e.g., heavy breathing) in the patient, the combination of information from different frames may result in blurred images or inaccurate information.

### SUMMARY

Described herein is a framework for characterizing contrast enhanced ultrasound (CEUS) images. The framework derives a brightness mode (B-mode) and contrast image sequence from the sequence of contrast enhanced ultrasound (CEUS) images. B-mode images and contrast images in the B-mode and contrast image sequence are spatially co-registered and temporally sub-sampled. A region of interest may be selected in a frame of the B-mode and contrast image sequence. The region of interest is then characterized based on the B-mode and contrast image sequence.

### BRIEF DESCRIPTION OF THE DRAWINGS

A more complete appreciation of the present disclosure and many of the attendant aspects thereof will be readily obtained as the same becomes better understood by reference to the following detailed description when considered in connection with the accompanying drawings.
FIG. 1 is a block diagram illustrating an exemplary imaging system;
FIG. 2 shows an exemplary method of characterization; and
FIG. 3 shows an exemplary timeline plot.

### DETAILED DESCRIPTION

In the following description, numerous specific details are set forth such as examples of specific components, devices, methods, etc., in order to provide a thorough understanding of implementations of the present framework. It will be apparent, however, to one skilled in the art that these specific details need not be employed to practice implementations of the present framework. In other instances, well-known materials or methods have not been described in detail in order to avoid unnecessarily obscuring implementations of the present framework. While the present framework is susceptible to various modifications and alternative forms, specific embodiments thereof are shown by way of example in the drawings and will herein be described in detail. It should be understood, however, that there is no intent to limit the invention to the particular forms disclosed, but on the contrary, the intention is to cover all modifications, equivalents, and alternatives falling within the spirit and scope of the invention. Furthermore, for ease of understanding, certain method steps are delineated as separate steps; however, these separately delineated steps should not be construed as necessarily order-dependent in their performance.

Unless stated otherwise as apparent from the following discussion, it will be appreciated that terms such as "segmenting," "generating," "registering," "determining," "aligning," "positioning," "processing," "computing," "selecting," "estimating," "detecting," "tracking" or the like may refer to the actions and processes of a computer system, or similar electronic computing device, that manipulates and transforms data represented as physical (e.g., electronic) quantities within the computer system's registers and memories into other data similarly represented as physical quantities within the computer system memories or registers or other such information storage, transmission or display devices. Embodiments of the methods described herein may be implemented using computer software. If written in a programming language conforming to a recognized standard, sequences of instructions designed to implement the methods can be compiled for execution on a variety of hardware platforms and for interface to a variety of operating systems. In addition, implementations of the present framework are not described with reference to any particular programming language. It will be appreciated that a variety of programming languages may be used.

One aspect of the present framework provides a tool to generate a summary of acquired data and allow physicians to review the data in a single static image timeline, instead of watching a video and observing separate images. Another aspect of the present framework derives a spatially co-registered image sequence of selected frames from a sequence of ultrasound images containing B-mode and contrast images. Both B-mode and contrast images are used to ensure reliable tracking of lesions throughout the contrast life-cycle, and compensate for motion (e.g., operator and/or internal motion) to facilitate accurate visualization and measurement. Using only B-mode images does not allow for accurate tracking, since B-mode images are affected by contrast wash-in and are therefore not reliable to use during tracking. These and other exemplary advantages and features will be described in more details in the following description.

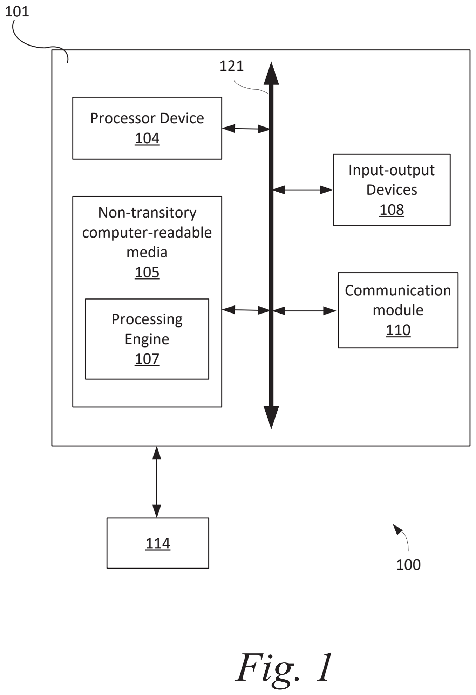

FIG. 1 is a block diagram illustrating an exemplary medical imaging system 100. In some implementations, system 100 includes a computer system 101 coupled to a medical imaging device 114. Computer system 101 includes a processor device 104 coupled to one or more non-transitory computer-readable media 105 (e.g., computer storage or memory device), input-output devices 108 (e.g., monitor, mouse, touchpad or keyboard) and communication module 110. Processor device 104 may include, for example, a central processing unit (CPU), a graphics processing unit (GPU), field-programmable gate array (FPGA), or a combination thereof. Computer system 101 may further include support circuits such as a cache, a power supply or battery, clock circuits, and a communications bus (not shown). Various other peripheral devices, such as client devices (e.g., workstations) and additional data storage devices and printing devices, may also be connected to the computer system 101.

The present technology may be implemented in various forms of hardware, software, firmware, special purpose processors, or a combination thereof, either as part of the microinstruction code or as part of an application program or software product, or a combination thereof, which is executed via the operating system. In some implementations, the techniques described herein are implemented as computer-readable program code tangibly embodied in one or more non-transitory computer-readable media 105. In particular, the present techniques may be implemented by a processing engine 107. Non-transitory computer-readable media 105 may include random access memory (RAM), read-only memory (ROM), magnetic floppy disk, flash memory, and other types of memories, or a combination thereof. The computer-readable program code is executed by processor device 104 to process data acquired by, for example, medical imaging device 114. The computer-readable program code is not intended to be limited to any particular programming language and implementation thereof. It will be appreciated that a variety of programming languages and coding thereof may be used to implement the teachings of the disclosure contained herein. The same or different computer-readable media 105 may be used for storing a database, including, but not limited to, image datasets, a knowledge base, individual subject data, medical records, diagnostic reports (or documents) for subjects, or a combination thereof.

Communication module 110 enables the computer system 101 to communicate with external systems and/or networks. In some implementations, communication module 110 includes a high-speed digital interface, such as Thunderbolt^{™}, universal serial bus (USB), multi-Gigabit Ethernet, optical fiber, waveguide technology, or a wireless interface. Other types of interfaces are also useful. In some implementations, communication module 110 includes wireless signal transceiver that communicates signals using a common communication protocol, such as Global System for Mobile Communications (GSM), WIFI, Bluetooth, Zigbee, LoRa, and TCP/IP.

Medical imaging device 114 is a radiological imaging device that acquires medical image data that reveals internal structures hidden by skin and bones of the subject. In some implementations, medical imaging device 114 is a contrast enhanced ultrasound (CEUS) system. The CEUS system acquires and displays B-mode images of tissue along with contrast images that are overlayed or displayed side-by-side with the B-mode images. The CEUS system may include a transmit beamformer, a transducer and a receive beamformer. Additional, different, or fewer components may be provided. For example, a separate memory may be provided for buffering or storing frames of data.

It is to be further understood that, because some of the constituent system components and method steps depicted in the accompanying figures can be implemented in software, the actual connections between the systems components (or the process steps) may differ depending upon the manner in which the present framework is programmed. Given the teachings provided herein, one of ordinary skill in the related art will be able to contemplate these and similar implementations or configurations of the present framework.

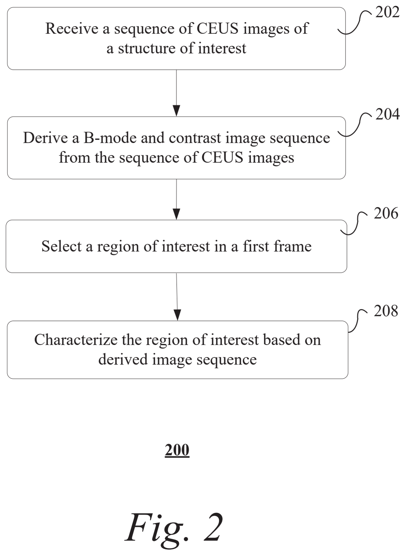

FIG. 2 shows an exemplary method 200 of characterization. It should be understood that the steps of the method 200 may be performed in the order shown or a different order. Additional, different, or fewer steps may also be provided. Further, the method 200 may be implemented with system 100 of FIG. 1, a different system, or a combination thereof.

At 202, processing engine 107 receives a sequence of contrast enhanced ultrasound (CEUS) images of at least one structure of interest. The sequence of CEUS images may be acquired by medical imaging device 114 (e.g., in real-time), or previously generated and retrieved from, for example, non-transitory computer-readable media 105. The sequence of CEUS images may be substantially continuous or periodic (e.g., acquired once or more every heart cycle). The CEUS sequence may be a video including frames of data representing a scanned region or structure of interest at different times after the administration of a contrast agent.

The structure of interest may be any anatomical structure (e.g., liver) that is identified for further study. The structure of interest includes a contrast agent or an area likely to include a contrast agent that has been administered. The contrast agent may respond to ultrasound energies. Some or all of the frames of the sequence include information from the contrast agent, as well as response from tissue or fluids.

In some implementations, the CEUS image sequence is acquired in two modes. Both B-mode images and contrast images are recorded simultaneously at, e.g., the same framerate and duration. B-mode images, also known as brightness mode images, are two-dimensional ultrasound images generated by translating the amplitude of echoes into varying shades of gray. Contrast images are two-dimensional color images that reflect the spatial distribution of contrast agents (e.g., microbubbles). Contrast images are formed by detecting and separating non-linear components of both linear and non-linear signals from the contrast agents in the tissue.

At 204, processing engine 107 derives a B-mode and contrast image sequence from the sequence of CEUS images. The B-mode and contrast image sequence includes a series of B-mode images and a corresponding series of contrast images extracted from the sequence of CEUS images. Each frame in the B-mode series corresponds to, and is spatially co-registered to, a frame in the contrast image series. In a concurrent mode, pairs of B-mode and contrast images may be concurrently acquired. In an alternating mode, pairs of B-mode and contrast images may be alternately acquired. Typically, the B-mode image is better quality in alternating mode, but the contrast and B-mode images may not be temporally aligned. The concurrent mode provides temporally aligned B-mode and contrast images.

B-mode images and contrast images in the derived image sequence are spatially co-registered and temporally sub-sampled. In some implementations, deriving the image sequence includes sub-sampling frames from the sequence of CEUS images that are evenly spaced in time. In other words, the time interval between the acquisition times of consecutive frames in each series is the same.

Alternatively, deriving the image sequence includes sub-sampling frames from the sequence of CEUS images that are unevenly spaced in time. In such implementation, particular frames are selected from the sequence of CEUS images to form the series of B-mode images and the series of contrast images. The frames may be selected for inclusion in each series based on at least one characteristic of the image data. Such characteristic includes, but is not limited to, motion displacement and similarity between frames. For example, frames associated with substantial motion and/or frames that are very similar to a previously selected frame are not selected for inclusion. Frames that are selected are "best" for quantification of tissue perfusion. The "best" frames are free of substantial motion (e.g., out-of-plane motion), and have sufficient signal intensity indicating adequate penetration and adequate sampling of temporal dynamics of the contrast agents. Out-of-plane motion cannot be compensated, while in-plane motion may be compensated using registration techniques.

Frames within each series may be spatially aligned to compensate for in-plane motion between frames. In-plane motion may be caused by transducer movement, patient movement, and/or movement of the tissue within the region of interest. To compensate for motion, a relative translation and/or rotation along one or more dimensions is determined. Data from one frame is correlated with different regions in the another frame of data to identify a best or sufficient match. Correlation, cross-correlation, minimum sum of absolute differences, and/or another measure of similarity may be used. Rigid or non-rigid motion models may be provided, such as warping in additional to translating and rotating in a non-rigid motion model. The entire frame of data or a window of data may be used for determining the best match and corresponding motion.

In some implementation, the extracted B-mode images and contrast images are spatially co-registered to compensate (or correct) for motion (e.g., in-plane motion). Unlike traditional methods, the present framework uses B-mode images in conjunction with the contrast images for motion correction. B-mode and/or contrast images may be used for motion correction based on the timing of the contrast administration. For example, only B-mode images acquired in the early wash-in phase may be used for motion correction as they provide a better representation of lesion borders, while only contrast images acquired in the late wash-out phase may be used for motion correction as they provide better information. Early wash-in generally refers to wash-in that is unequivocally detectable earlier than a pre-determined time (e.g., 60 seconds) after contrast administration. Late wash-out generally refers to wash-out that is unequivocally detectable at the pre-determined time (e.g., 60 seconds) or later after contrast administration. Alternatively, both B-mode and contrast images are used for motion correction if the lesion-to-liver contrast is high and the B-mode images are not affected by contrast fill-in.

At 206, processing engine 107 selects at least one region of interest in a first frame of the series of B-mode images and/or series of contrast images. The region of interest in the first frame may be selected by an operator via, for example, a user interface. Alternatively, the region of interest may be semi-automatically or automatically detected in the first frame by, for example, a machine learning technique. The region of interest may correspond to a lesion or other abnormality in the structure of interest. More than one region of interest (e.g., multiple lesions) may be selected.

At 208, processing engine 107 characterizes the region of interest based on the derived image sequence. The output of such characterization may be, for example, qualitative parameters of the region of interest, quantitative parameters of the region of interest, a timeline plot, or a combination thereof. The characterization output may be presented (or displayed) in, for example, a user interface at a user device to facilitate diagnosis. The characterization output may also be stored in, for example, an electronic health record to be accessed by, for instance, the patient and healthcare providers.

To generate qualitative parameters, the derived image sequence may be input into a diagnostic function module. The B-mode image series may be used to assess, for example, the size, volume, shape, echogenicity and arrangement of the region of interest, while the contrast image series may be used to assess, for example, blood flow dynamics and vascularization pattern, such as arterial phase contrast wash-in characteristics (e.g., filling pattern, dynamics, timing) and portal phase wash-out characteristics (e.g., degree of wash-out, timing). Additionally, the characteristics may further include the type of lesion (e.g., benign or malignant). The diagnostic function module may implement a deep-learning technique or any other suitable techniques to, for example, extract the contrast characteristics or to predict the lesion type (e.g., malignant, benign), lesion class (e.g., hepatocellular carcinoma or HCC, focal nodular hyperplasia or FNH, hemangioma), or Liver Imaging Reporting and Data System (LI-RADS) category.

In some implementations, processing engine 107 performs quantification of the region of interest based on the derived image sequence to generate quantitative parameters. Quantification may be performed by extracting time-intensity ratio curves between the calculated lesion and liver intensities or using deep learning-based techniques. Such curves may provide information about contrast wash-in and wash-out characteristics, as previously mentioned. Processing engine 107 may select the "best" frames from each of the series for quantification using, for example, similarity measurements to a reference frame (e.g., first frame) or deep learning-based techniques used in object tracking.

In some implementations, processing engine 107 generates a timeline plot from the derived image sequence that tracks the region of interest. The timeline plot is a visual representation of the series of B-mode images adjacent to (e.g., above or below) the series of contrast images progressing over time. The timeline plot may provide a single static representation of characteristics of the region-of-interest, such as Arterial Phase (AP) wash-in pattern (e.g., centrifugal, centripetal, rim-like, globular), wash-out timing, and so forth.

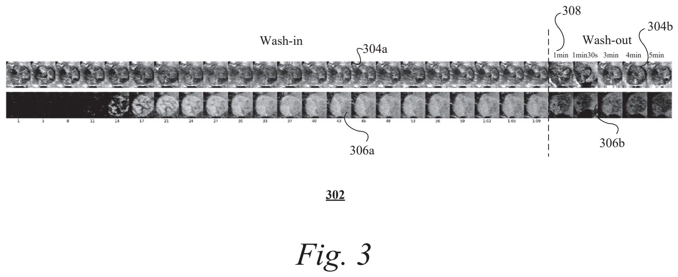

FIG. 3 shows an exemplary timeline plot 302. The timeline plot 302 includes a series of B-mode images 304a-b displayed above a series of contrast images 306a-b. Although a static horizontal timeline plot 302 is shown, it should be appreciated that other configurations (e.g., vertical) of the timeline plot are also possible. The timeline plot 302 includes both Arterial Phase (AP) wash-in images (304a, 306a). The AP wash-in images (304a, 306a) are finely sub-sampled and preselected. Additionally, Portal Vein Phase wash-out images (304b, 306b) may also be included. The Portal Vein Phase wash-out images (304b, 306b) may be sparsely sampled. The acquisition time 308 may be displayed above the timeline plot 302.

Additional timeline plots may be generated to represent additional regions of interest or multiple contrast agent administrations for a single region of interest. For example, there may be multiple lesions in one person's liver or multiple contrast agent injections administered for the visualization of a single lesion. Multiple timeline plots may be shown to the user to provide additional information about contrast behavior. Multiple timeline plots for multiple lesions may be shown concurrently to facilitate visual comparison of the lesions.

The following is a list of non-limiting illustrative embodiments disclosed herein:
Illustrative embodiment 1. A method of image characterization, comprising: receiving a sequence of contrast enhanced ultrasound (CEUS) images; deriving a brightness mode (B-mode) and contrast image sequence from the sequence of CEUS images, wherein B-mode images and contrast images in the B-mode and contrast image sequence are spatially co-registered and temporally sub-sampled; selecting at least one region of interest in the B-mode and contrast image sequence; and characterizing the at least one region of interest based on the B-mode and contrast image sequence.
Illustrative embodiment 2. The method of illustrative embodiment 1 wherein deriving the B-mode and contrast image sequence comprises sub-sampling frames that are evenly spaced in time.
Illustrative embodiment 3. The method of any one of illustrative embodiments 1-2 wherein deriving the B-mode and contrast image sequence comprises sub-sampling frames comprises sub-sampling frames that are unevenly spaced in time.
Illustrative embodiment 4. The method of illustrative embodiment 3 wherein the frames are selected from the sequence of CEUS images based on motion displacement, similarity between frames, or a combination thereof.
Illustrative embodiment 5. The method of any one of illustrative embodiments 1-4 wherein deriving the B-mode and contrast image sequence comprises spatially co-registering B-mode and contrast images to correct for motion.
Illustrative embodiment 6. The method of illustrative embodiment 5 wherein only B-mode images acquired in early wash-in phase and only contrast images acquired in late wash-out phase are used for motion correction.
Illustrative embodiment 7. The method of illustrative embodiment 5 wherein both B-mode images and contrast images are used for motion correction.
Illustrative embodiment 8. The method of any one of illustrative embodiments 1-7 wherein characterizing the at least one region of interest based on the B-mode and contrast image sequence comprises generating one or more qualitative parameters.
Illustrative embodiment 9. The method of any one of illustrative embodiments 1-8 wherein characterizing the at least one region of interest based on the B-mode and contrast image sequence comprises generating one or more quantitative parameters.
Illustrative embodiment 10. The method of any one of illustrative embodiments 1-9 wherein characterizing the at least one region of interest based on the B-mode and contrast image sequence comprises generating a timeline plot.
Illustrative embodiment 11. The method of illustrative embodiment 10 wherein generating the timeline plot comprises generating a static horizontal timeline plot.
Illustrative embodiment 12. The method of any one of illustrative embodiments 1-11 wherein characterizing the at least one region of interest based on the B-mode and contrast image sequence comprises generating multiple timeline plots for multiple contrast agent administrations for the region of interest.
Illustrative embodiment 13. An image processing system, comprising: a non-transitory memory device for storing computer readable program code; and a processor device in communication with the non-transitory memory device, the processor device being operative with the computer readable program code to perform steps including deriving a brightness mode (B-mode) and contrast image sequence from a sequence of contrast enhanced ultrasound (CEUS) images, wherein B-mode images and contrast images in the B-mode and contrast image sequence are spatially co-registered and temporally sub-sampled, selecting at least one region of interest in the B-mode and contrast image sequence, and characterizing the at least one region of interest based on the B-mode and contrast image sequence.
Illustrative embodiment 14. The image processing system of illustrative embodiment 13 wherein the processor device is operative with the computer readable program code to derive the B-mode and contrast image sequence by sub-sampling frames that are evenly spaced in time.
Illustrative embodiment 15. The image processing system of any one of illustrative embodiments 13-14 wherein the processor device is operative with the computer readable program code to derive the B-mode and contrast image sequence by sub-sampling frames that are unevenly spaced in time.
Illustrative embodiment 16. The image processing system of any one of illustrative embodiments 13-15 wherein the processor device is operative with the computer readable program code to select the at least one region of interest by selecting one or more lesions.
Illustrative embodiment 17. The image processing system of any one of illustrative embodiments 13-16 wherein the processor device is operative with the computer readable program code to characterize the at least one region of interest based on the B-mode and contrast image sequence by generating one or more qualitative parameters, quantitative parameters, timeline plots, or a combination thereof.
Illustrative embodiment 18. The image processing system of any one of illustrative embodiments 13-17 wherein the processor device is operative with the computer readable program code to characterize the at least one region of interest based on the B-mode and contrast image sequence by generating a static horizontal timeline plot.
Illustrative embodiment 19. The image processing system of any one of illustrative embodiments 13-18 wherein the processor device is operative with the computer readable program code to characterize the at least one region of interest based on the B-mode and contrast image sequence by generating multiple timeline plots for multiple contrast agent administrations for the region of interest.
Illustrative embodiment 20. One or more non-transitory computer-readable media embodying instructions executable by a machine to perform operations comprising: deriving a brightness mode (B-mode) and contrast image sequence from a sequence of contrast enhanced ultrasound (CEUS) images, wherein B-mode images and contrast images in the B-mode and contrast image sequence are spatially co-registered and temporally sub-sampled; selecting at least one region of interest in the B-mode and contrast image sequence; and characterizing the at least one region of interest based on the B-mode and contrast image sequence.

While the present framework has been described in detail with reference to exemplary embodiments, those skilled in the art will appreciate that various modifications and substitutions can be made thereto without departing from the spirit and scope of the invention as set forth in the appended claims. For example, elements and/or features of different exemplary embodiments may be combined with each other and/or substituted for each other within the scope of this disclosure and appended claims.

## Claims

1. A method (200) of image characterization, comprising:
receiving (202) a sequence of contrast enhanced ultrasound, CEUS, images;
deriving (204) a brightness mode, B-mode, and contrast image sequence from the sequence of CEUS images, wherein B-mode images and contrast images in the B-mode and contrast image sequence are spatially co-registered and temporally sub-sampled;
selecting (206) at least one region of interest in the B-mode and contrast image sequence; and
characterizing (208) the at least one region of interest based on the B-mode and contrast image sequence.

2. The method (200) of claim 1 wherein deriving (204) the B-mode and contrast image sequence comprises sub-sampling frames that are evenly spaced in time.

3. The method (200) of claim 1 wherein deriving (204) the B-mode and contrast image sequence comprises sub-sampling frames comprises sub-sampling frames that are unevenly spaced in time.

4. The method (200) of claim 3 wherein the frames are selected from the sequence of CEUS images based on motion displacement, similarity between frames, or a combination thereof.

5. The method (200) of one of claims 1 to 4 wherein deriving (204) the B-mode and contrast image sequence comprises spatially co-registering B-mode and contrast images to correct for motion.

6. The method (200) of claim 5 wherein only B-mode images acquired in early wash-in phase and only contrast images acquired in late wash-out phase are used for motion correction.

7. The method (200) of claim 5 wherein both B-mode images and contrast images are used for motion correction.

8. The method (200) of one of claims 1 to 7 wherein characterizing (208) the at least one region of interest based on the B-mode and contrast image sequence comprises generating one or more qualitative parameters.

9. The method (200) of one of claims 1 to 8 wherein characterizing (208) the at least one region of interest based on the B-mode and contrast image sequence comprises generating one or more quantitative parameters.

10. The method (200) of one of claims 1 to 9 wherein characterizing (208) the at least one region of interest based on the B-mode and contrast image sequence comprises generating a timeline plot.

11. The method (200) of claim 10 wherein generating the timeline plot comprises generating a static horizontal timeline plot (302).

12. The method (200) of one of claims 1 to 11 wherein characterizing (208) the at least one region of interest based on the B-mode and contrast image sequence comprises generating multiple timeline plots for multiple contrast agent administrations for the region of interest.

13. An image processing system (100), comprising:
a non-transitory memory device (105) for storing computer readable program code; and
a processor device (104) in communication with the non-transitory memory device (105), the processor device (104) being operative with the computer readable program code to perform steps including
deriving (204) a brightness mode, B-mode, and contrast image sequence from a sequence of contrast enhanced ultrasound, CEUS, images, wherein B-mode images and contrast images in the B-mode and contrast image sequence are spatially co-registered and temporally sub-sampled,
selecting (206) at least one region of interest in the B-mode and contrast image sequence, and
characterizing (208) the at least one region of interest based on the B-mode and contrast image sequence.

14. The image processing system (100) of claim 13 wherein the processor device (104) is operative with the computer readable program code to characterize (208) the at least one region of interest based on the B-mode and contrast image sequence by generating one or more qualitative parameters, quantitative parameters, timeline plots, or a combination thereof.

15. The image processing system (100) of claim 13 or 14 wherein the processor device (104) is operative with the computer readable program code to characterize (208) the at least one region of interest based on the B-mode and contrast image sequence by generating a static horizontal timeline plot (302).
